# EUROPÄISCHE PATENTSCHRIFT

(11) **EP 1 256 324 B1**
(45) Veröffentlichungstag und Bekanntmachung des Hinweises auf die Patenterteilung: **10.03.2004**
(21) Anmeldenummer: 02008626.0
(22) Anmeldetag: 17.04.2002
(51) Int. Cl.: A61B 17/34, A61N 1/05

(54) **Punktionskanüle**
Hollow puncturing needle
Canule pour ponction

(30) Priorität: 08.05.2001 DE 20107778 U
(43) Veröffentlichungstag der Anmeldung: 13.11.2002
(73) Patentinhaber: B. BRAUN MELSUNGEN AG, 34212 Melsungen (DE)
(72) Erfinder: Freigang, Helmut, 34327 Körle (DE); Seeber, Manfred, 34587 Felsberg (DE); Siemon, Klaus, 34327 Körle (DE)
(74) Vertreter: Selting, Günther, Dipl.-Ing.

(56) Entgegenhaltungen:
- EP-A- 0 244 017
- DE-U- 29 803 118

## Beschreibung

Die Erfindung betrifft eine Punktionskanüle, insbesondere für die Nervenstimulation, mit einer Stahlkanüle, die an einem Kanülenansatz befestigt ist, und einem durch einen Einführkanal verlaufenden Kabel, dessen Ader mit der Stahlkanüle verbunden ist.

Aus EP 0 102 538 B1 ist eine Punktier- und Katheterisierungsvorrichtung bekannt, die zum Punktieren und Katheterisieren von Nervensträngen geeignet ist. Diese Vorrichtung weist eine Stahlkanüle mit einem am distalen Ende vorgesehenen Kanülenansatz auf, wobei die Stahlkanüle mit einem Kabel verbunden ist, über das ein elektrisches Potential an die Stahlkanüle angelegt werden kann. Wenn die Stahlkanüle, die eine blanke Spitze aufweist, in die Nähe eines Nervs gelangt, erfolgt durch elektrische Impuls, die durch ein entsprechendes Stimulationsgerät an das Kabel angelegt werden, eine Nervenstimulation mit entsprechenden Reflexen des Patienten. Auf diese Weise ist feststellbar, ob die Kanülenspitze hinreichend nahe an den gewünschten Nerv herangeführt ist. Anschließend kann durch die Kanüle oder durch einen mit Hilfe der Kanüle verlegten Katheter ein Anästhetikum zugespritzt werden, um eine Lokalanästhesie durchzuführen. Bei der bekannten Vorrichtung erfolgt das elektrische Verbinden der Ader des Kabels mit der Stahlkanüle durch Umwickeln oder durch Anlöten und anschließendes Umspritzen. Eine solche Verbindungstechnik ist kompliziert und mit hohen Kosten verbunden.

DE 29 803 118 offenbart eine Punktionskanüle nach den Oberbegriff des Anspruchs 1.

Der Erfindung liegt die Aufgabe zugrunde, eine Punktionskanüle zu schaffen, die einfach herstellbar ist und einen sicheren Kontakt zwischen Kabel und Punktionskanüle gewährleistet.

Die Lösung dieser Aufgabe erfolgt erfindungsgemäß mit den im Anspruch 1 angegebenen Merkmalen. Hiernach enthält der Kanülenansatz ein metallisches Klemmelement, welches einen an der Stahlkanüle angreifenden ersten Klemmschlitz und einen an der Kabelader angreifenden zweiten Klemmschlitz aufweist. Durch Einschieben des Klemmelements in den Kanülenansatz gelangen die Stahlkanüle und die Kabelader automatisch in den jeweiligen Klemmschlitz, der sie eng umschließt. Auf diese Weise erfolgt ein gleichzeitiges Festklemmen der beiden genannten Teile, ohne dass die Klemmung des einen Teils durch das andere Teil beeinträchtigt wird. Das Klemmelement ist ein einstückiges Klemmelement, dessen Klemmschlitze so angeordnet sind, dass der erste Klemmschlitz die Stahlkanüle einklemmt, wenn der zweite Klemmschlitz, die Kabelader einklemmt. Die Klemmschlitze haben also den gleichen Abstand wie Stahlkanüle und Kabelader und sie werden durch Verschieben des Klemmelements wirksam.

Ein wesentlicher Vorteil der Erfindung besteht in der einfachen und sicheren Montage. Dabei werden Stahlkanüle und Kabel durch Verschieben des Klemmelements relativ zueinander fixiert und auch in Bezug auf den Kanülenansatz fixiert. Es ist möglich, die Klemmung vorzunehmen bevor die Stahlkanüle im Kanülenansatz befestigt worden ist. Hierbei wird nach dem Einschieben des Klemmelements ein Kleber in eine Ausnehmung des Kanülenansatzes eingefüllt. Dieser Kleber dringt in sämtliche Spalte ein und befestigt dadurch auch die Stahlkanüle am Kanülenansatz. Außerdem wird das Kabel von dem Kleber abgeschirmt umschlossen und zusätzlich fixiert. Eine weitere Funktion des Klebers besteht darin, dass er sämtliche metallischen Teile, die am oder im Kanülenansatz vorhanden sind, bedeckt, so dass nicht die Möglichkeit besteht, dass eine Person unabsichtlich mit einem der stromführenden Teile in Berührung kommt.

Gemäß einer bevorzugten Ausgestaltung der Erfindung ist vorgesehen, dass der zweite Klemmschlitz schneidende Kanten aufweist, die eine Isolierung des Kabels durchdringen. Hierbei ist es nicht erforderlich, zuvor eine Abisolierung des Kabels vorzunehmen und die Kabelader freizulegen. Beim Einschieben des Klemmelements in den Kanülenansatz erfolgt automatisch das Durchtrennen der Kabelisolierung und das Klemmen der Kabelader.

Vorzugsweise sind die Klemmschlitze in der Platte hintereinander angeordnet, wobei zwischen ihnen eine verbreiterte Öffnung vorgesehen ist. Durch die Öffnung werden die beiden Klemmschlitze entkoppelt.

Die erfindungsgemäße Punktionskanüle eignet sich besonders für die Nervenstimulation, wobei unterschiedliche Kanülentechniken anwendbar sind. So ist es beispielsweise möglich, ein Anästhetikum unmittelbar durch die Stahlkanüle hindurch zu injizieren oder die Stahlkanüle mit einem Kurzkatheter oder Kapillar zu verbinden. Es besteht auch die Möglichkeit der Verlegung eines Katheters, durch den von der Stahlkanüle geschaffenen Punktionskanal, entweder mit oder ohne Führungsdraht.

Das Klemmelement kann derart ausgestaltet sein, dass es für unterschiedliche Durchmesser von Stahlkanülen und/oder Kabeladern geeignet ist.

Im folgenden wird unter Bezugnahme auf die Zeichnungen ein Ausführungsbeispiel der Erfindung näher erläutert.

Es zeigen:
- Fig. 1: einen Längsschnitt durch eine Ausführungsform der Punktionskanüle,
- Fig. 2: einen Schnitt entlang der Linie II-II von Fig. 1, und
- Fig. 3: eine Ansicht aus Richtung des Pfeiles III von Fig. 1.

Die Punktionskanüle weist eine langgestreckte hohle Stahlkanüle 10 auf, die an einem Kanülenansatz 11 aus Kunststoff befestigt ist. Die Stahlkanüle 10 besteht aus einem Rohr, das mit einer nicht leitenden Beschichtung 12 versehen ist, wobei die (nicht dargestellte) Kanülenspitze freiliegt.

Auf einem Rohrstutzen 13 des Kanülenansatzes 11 sitzt ein Schutzschlauch 9, der die Stahlkanüle 10 umgibt und in der Länge überragt, um Verletzungen durch die Kanülenspitze zu vermeiden. Der Schutzschlauch 9 kann von dem Rohrstutzen 13 abgezogen werden.

Der distale Bereich 10a der Stahlkanüle 10 verläuft durch einen Hohlraum bzw. eine Ausnehmung 14 des Kanülenansatzes hindurch und er endet in einem Hohlraum 15, der durch einen Schlauchkonnektor 16 abgeschlossen ist. Von dem Schlauchkonnektor 16 erstreckt sich ein Schlauch 17, durch den ein Anästhetikum zugeführt werden kann, das dann durch die Stahlkanüle hindurch in den Patientenkörper injiziert wird.

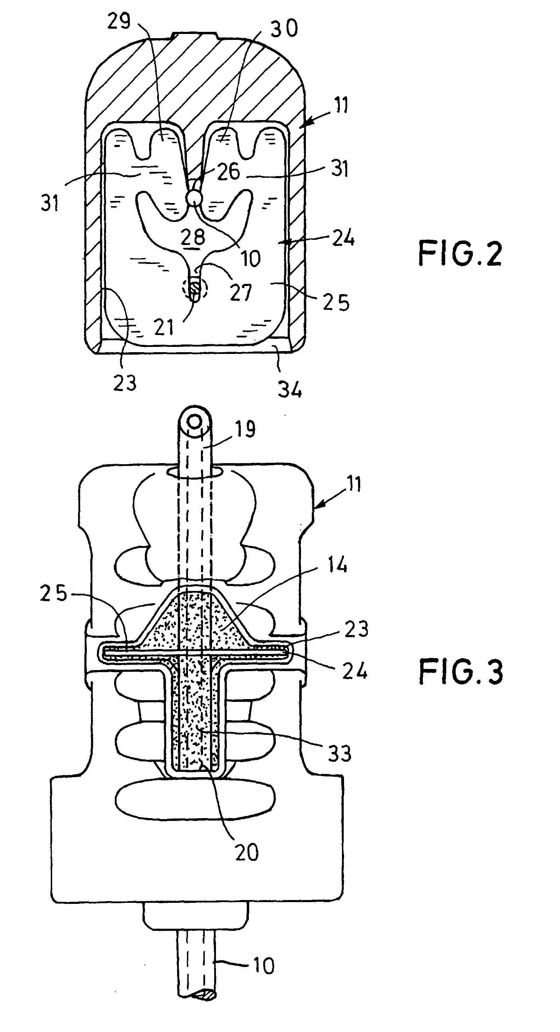

In die Ausnehmung 14 führt ferner Einführkanal 18, durch den ein Kabel 19 hindurchgesteckt ist. Der Einführkanal 18 ist auf einem Teil 18a seiner Länge als umfangsmäßig geschlossene Bohrung ausgebildet und auf einem anderen Teil 18b seiner Länge als offener Kanal. Der Teil 18b endet an einer Endwand 20, die einen Anschlag für das Ende des Kabels 19 bildet. Das Kabel 19 weist eine aus Kupfer bestehende Kabelader 21 und eine Isolierung 22 auf. Der Einführkanal 18 verläuft unter einem spitzen Winkel zur Längsachse der Stahlkanüle 10. Quer zur Stahlkanüle 10 und zu dem Kabel 19 verläuft eine Führung 23, in der ein Klemmelement 24 bewegbar ist. Das Klemmelement 24 besteht aus einer elastischen Platte 25 aus Federstahl. In dieser Platte sind gemäß Fig. 2 ein erster Klemmschlitz 26 für die Stahlkanüle 10 und ein zweiter Klemmschlitz 27 für die Kabelader 21 ausgebildet. Beide Klemmschlitze liegen hintereinander entlang einer gemeinsamen Achse und zwischen ihnen befindet sich eine verbreiterte Öffnung 28. Jeder der Klemmschlitze 26 und 27 verjüngt sich in Einsteckrichtung, also gemäß Fig. 2 von oben nach unten, so dass mit zunehmenden Einschieben des Klemmelements 24 in den Kanülenansatz 11 die Stahlkanüle 10 bzw. die Kabelader 21 verstärkt festgeklemmt wird. Die Kanten des zweiten Klemmschlitzes 27 sind schneidende Kanten, die die Isolierung 22 des Kabels 19 durchschneiden und einen sicheren elektrischen Kontakt zur Kabelader 21 herstellen.

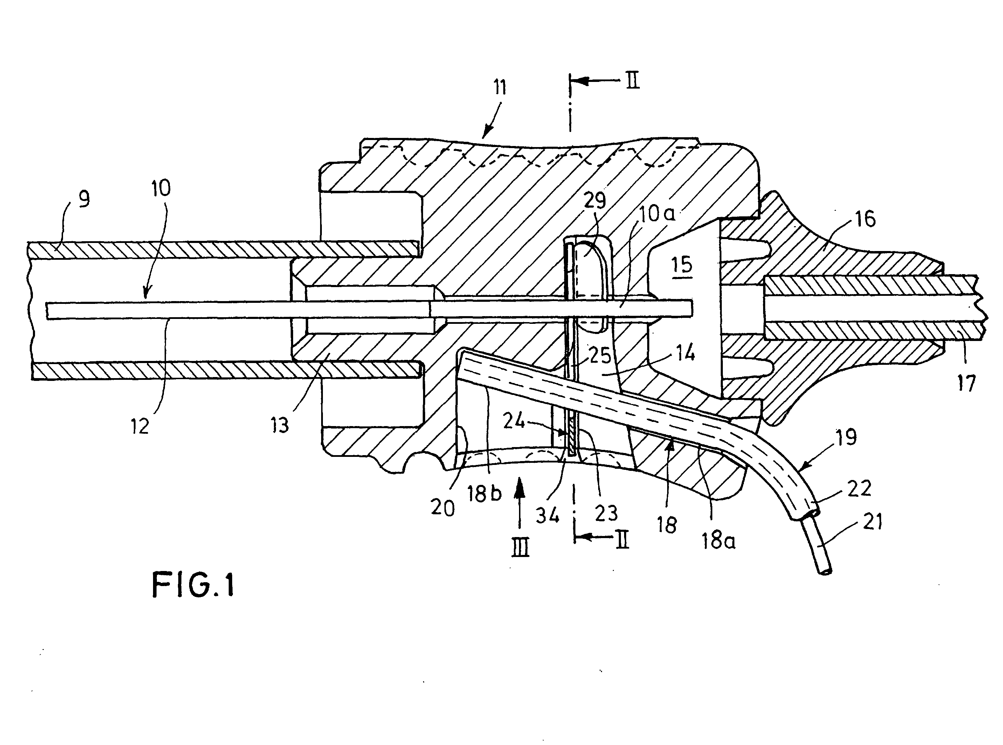

Der Klemmschlitz 26 wird von Flügeln 29,30 begrenzt, die um Biegestellen 31 herum gebogen werden können, um sich unterschiedlichen Durchmessern von Stahlkanülen 10 anzupassen. Ein Flügel 29 ist im ausgebogenen Zustand in Fig. 1 dargestellt.

Das Klemmelement 24 besteht aus einer Platte, die jedoch nicht notwendigerweise flach sein muss. Die Platte ist in einer linearen Führung 23 geführt und kann zwischen einer Klemmposition und einer Freigabeposition verschoben werden.

Bei der Montage von Stahlkanüle 10 und Kabel 19 wird die Stahlkanüle in den dafür vorgesehenen Kanal des Kanülenansatzes 11 eingesetzt und das Kabel 19 mit Isolierung 22 wird in den Einführkanal 18 eingeführt. Dann wird das Klemmelement 24 in die Führung 23 eingeschoben und bis zum Endanschlag eingedrückt, wobei der Klemmschlitz 26 die Stahlkanüle 10 einklemmt und der Klemmschlitz 27 durch die Isolierung 22 hindurch in die Kabelader 21 einschneidet. Der Kanülenansatz 11 wird dann so positioniert, dass die Ausnehmung 14 nach oben zeigt. In diesem Zustand würde Fig. 3 die Draufsicht darstellen. In die Ausnehmung 14 wird ein flüssiger Kleber 33 eingefüllt. Dieser Kleber füllt die Ausnehmung 14 vollständig aus. Er fixiert nach dem Erhärten auch das Klemmelement 24 und er dringt in den Ringspalt zwischen Stahlkanüle 10 und Kanülenansatz 11 ein sowie in den Ringspalt zwischen dem Einführkanal 18 und dem Kanülenansatz. Durch einen einzigen Klebevorgang werden also sämtliche Teile relativ zueinander fixiert und abdichtend verschlossen. Auch der Einlass 34 der Führung 23 wird mit Kleber verschlossen und abgedichtet, so dass das gesamte Klemmelement 24 in Kleber eingebettet ist. Somit sind keine elektrisch leitenden Teile von außen zugänglich.

## Patentansprüche

1. Punktionskanüle, insbesondere für die Nervenstimulation, mit einer Stahlkanüle (10), die an einem Kanülenansatz (11) befestigt ist, und einem durch einen Einführkanal (18) verlaufenden Kabel (19), dessen Ader (21) mit der Stahlkanüle (10) verbunden ist,
**dadurch gekennzeichnet ,**
**dass** der Kanülenansatz (11) ein metallisches Klemmelement (24) enthält, welches einen an der Stahlkanüle (10) angreifenden ersten Klemmschlitz (26) und einen an der Kabelader (21) angreifenden zweiten Klemmschlitz (27) aufweist.

2. Punktionskanüle nach Anspruch 1, **dadurch gekennzeichnet, dass** das Klemmelement (24) aus einer relativ zum Kanülenansatz (11) quer zur Längsrichtung der Stahlkanüle (10) verschiebbaren Platte (25) besteht, die in den Kanülenansatz (11) einschiebbar ist und im eingeschobenen Zustand gegen Herausziehen fixiert ist.

3. Punktionskanüle nach Anspruch 1 oder 2, **dadurch gekennzeichnet, dass** der zweite Klemmschlitz (27) schneidende Kanten aufweist, die eine Isolierung (22) des Kabels (19) durchdringen.

4. Punktionskanüle nach Anspruch 2, **dadurch gekennzeichnet, dass** die Klemmschlitze (26,27) in der Platte (25) hintereinander angeordnet sind, wobei zwischen ihnen eine verbreiterte Öffnung (28) vorgesehen ist.

5. Punktionskanüle nach einem der Ansprüche 1-4, **dadurch gekennzeichnet, dass** der Einführkanal (18) mindestens auf einem Teil (18a) seiner Länge als Bohrung ausgebildet ist.

6. Punktionskanüle nach einem der Ansprüche 1-5, **dadurch gekennzeichnet, dass** der Kanülenansatz (11) eine Ausnehmung (14) aufweist, durch die die Stahlkanüle (10) und ein Abschnitt des Kabels (19) hindurchgehen und dass in der Ausnehmung (14) eine Führung (23) zum Verschieben des Klemmelements (24) vorgesehen ist.

7. Punktionskanüle nach Anspruch 6, **dadurch gekennzeichnet, dass** die Ausnehmung (14) mit einem Kleber (33) gefüllt ist, der auch das Klemmelement (24) bedeckt.

## Claims

1. A puncture cannula, particularly for nerve stimulation, comprising a steel cannula (10) attached to a cannula hub (11), and a cable (19) extending through an insertion channel (18) and having a core (21) connected to the steel cannula (10),
**characterized in**
**that** the cannula hub (11) comprises a metallic clamping element (24) having a first clamping slot (26) for engaging the steel cannula (10) and a second clamping slot (27) for engaging the core (21) of the cable.

2. The puncture cannula according to claim 1, **characterized in that** the clamping element (24) comprises a plate (25), arranged for displacement relative to the cannula hub (11) in a direction transverse to the longitudinal direction of the steel cannula (10), which plate (25) is adapted to be inserted into the cannula hub (11) and in the inserted condition is fixed against withdrawal.

3. The puncture cannula according to claim 1 or 2, **characterized in that** the second clamping slot (27) has cutting edges for penetrating an insulation (22) of the cable (19).

4. The puncture cannula according to claim 2, **characterized in that** the clamping slots (26,27) are located behind each other in the plate (25) and have a widened opening (28) arranged between them.

5. The puncture cannula according to any one of claims 1-4, **characterized in that** the insertion channel (18) is at least along a part (18a) of its length formed as a bore.

6. The puncture cannula according to any one of claims 1-5, **characterized in that** the cannula hub (11) comprises a recess (14) having the steel cannula (10) and a portion of the cable (19) passing therethrough, and that the recess (14) is provided with a guideway (23) for displacement of the clamping element (24).

7. The puncture cannula according to claim 6, **characterized in that** the recess (14) is filled with adhesive (33) covering also the clamping element (24).

## Revendications

1. Canule pour ponction, notamment pour la stimulation des nerfs, comprenant une canule en acier (10) fixée à un raccord de canule (11), et un câble (19) s'étendant dans un canal d'introduction (18) et dont le conducteur (21) est relié à la canule en acier (10), **caractérisée en ce que**
le raccord de canule (11) contient un élément de serrage (24) métallique, qui présente une première fente de serrage (26) mettant en prise la canule en acier (10) et une deuxième fente de serrage (27) mettant en prise le conducteur de câble (21).

2. Canule pour ponction selon la revendication 1, **caractérisée en ce que** l'élément de serrage (24) comprend une plaque (25) pouvant être déplacée par rapport au raccord de canule (11) dans le sens transversal à la direction longitudinale de la canule en acier (10) et pouvant être insérée dans le raccord de canule (11) et qui, à l'état inséré, est fixée de manière à ne pas pouvoir être retirée.

3. Canule pour ponction selon la revendication 1 ou 2, **caractérisée en ce que** la deuxième fente de serrage (27) présente des arêtes coupantes qui pénètrent dans une isolation (22) du câble (19).

4. Canule pour ponction selon la revendication 2, **caractérisée en ce que** les fentes de serrage (26, 27) sont disposées l'une derrière l'autre dans la plaque (25), un orifice (28) étendu étant prévu entre les deux.

5. Canule pour ponction selon l'une des revendications 1 à 4, **caractérisée en ce que** le canal d'introduction (18) est formé au moins sur une partie (18a)de sa longueur en tant que perçage.

6. Canule pour ponction selon l'une des revendications 1 à 5, **caractérisée en ce que** le raccord de canule (11) présente un évidement (14) traversé par la canule en acier (10) et une partie du câble (19), et **en ce qu'**un guidage (23) est prévu à l'intérieur de l'évidement (14) pour déplacer l'élément de serrage (24).

7. Canule pour ponction selon la revendication 6, **caractérisée en ce que** l'évidement (14) est rempli d'une colle (33) qui recouvre également l'élément de serrage (24).
